# EUROPÄISCHE PATENTSCHRIFT

(11) **EP 0 925 785 B1**
(45) Veröffentlichungstag und Bekanntmachung des Hinweises auf die Patenterteilung: **27.03.2002**
(21) Anmeldenummer: 98123663.1
(22) Anmeldetag: 11.12.1998
(51) Int. Cl.: A61K 9/08, A61K 47/18

(54) **Stabilisierte pharmazeutische Zusammensetzung enthaltend Dopamin oder Dobutamin**
Stabilised pharmaceutical compositions containing dopamine or dobutamine
Compositions pharmaceutiques stabilisées contenant de la dopamine ou de la dobutamine

(30) Priorität: 19.12.1997 DE 19756758
(43) Veröffentlichungstag der Anmeldung: 30.06.1999
(73) Patentinhaber: Solvay Pharmaceuticals GmbH, 30173 Hannover (DE)
(72) Erfinder: Bonnacker, Ingo, Dr., 31171 Nordstemmen (DE); Tölke, Wolfgang, 30900 Wedemark (DE); Lerch, Martina, 30855 Langenhagen (DE)
(74) Vertreter: Gosmann, Martin

(56) Entgegenhaltungen:
- WO-A-94/13274
- DE-A- 2 052 991
- US-A- 5 002 973
- DATABASE WPI Section Ch, Week 8402 Derwent Publications Ltd., London, GB; Class B05, AN 84-008641 XP002093002 & JP 58 203910 A (NIKKEN CHEM KK) , 28. November 1983
- DATABASE WPI Section Ch, Week 8301 Derwent Publications Ltd., London, GB; Class B05, AN 83-01093K XP002093003 & JP 57 188515 A (KYOTO YAKUHIN KOGYO KK) , 19. November 1982

## Beschreibung

Die vorliegende Erfindung betrifft neue stabilisierte wäßrige flüssige pharmazeutische Zusammensetzungen, die als Wirkstoff Dopamin oder Dobutamin enthalten und sich für Injektionszwecke eignen.

Es ist im Stand der Technik bekannt, daß die Catecholamine Dopamin und Dobutamin, z.B. in Form ihrer Hydrochloride, als solche in wäßrigen Lösungen nicht stabil sind. Die Zersetzungsprodukte sind zwar bisher noch nicht indentifiziert worden, jedoch werden als typische Zersetzungserscheinungen üblicherweise Verfärbungen der wäßrigen Zubereitung bzw. gegebenenfalls sogar Niederschläge beobachtet. Wäßrige Lösungen von Dopamin bzw. Dobutamin, die als Injektionslösungen verwendet werden sollen, müssen daher in geeigneter Weise stabilisiert werden, um einerseits die erforderliche Hitzesterilisation bei der Zubereitung der Injektionslösung zu überstehen und um andererseits eine ausreichende Lagerfähigkgeit der Injektionslösung zu gewährleisten.

Im Stand der Technik war es bisher allgemein üblich, wäßrige Lösungen von Dopamin oder Dobutamin durch Zugabe von Sulfiten, beispielsweise durch Zugabe von Natriummetabisulfit, zu stabilisieren. Die Verwendung von Sulfiten kann unter ungünstigen Bedingungen jedoch beim Menschen zu Problemen führen, beispielsweise im Hinblick auf ungenügende Verträglichkeit und unerwünschte Toxizität. Die pharmazeutische Industrie sucht daher seit längerem, wäßrige Lösungen von Dopamin oder Dobutamin bereitzustellen, die ausreichend stabil sind, um eine Hitzesterilisation durchführen zu können bzw. die eine ausreichende Konservierung aufweisen, um auch längere Lagerzeiträume unbeschadet zu überstehen.

Zur Vermeidung der vorstehenden Probleme wurde daher für Dobutamin in der internationalen Patentanmeldung WO 97/15329 vorgeschlagen, Dobutamin enthaltende, wäßrige pharmazeutische Zubereitungen durch Zugabe von Ascorbinsäure zu stabilisieren. Die Verwendung von Ascorbinsäure in wäßrigen Lösungen erfordert jedoch gegebenenfalls zusätzliche Maßnahmen im Hinblick auf die Stabilisierung (Pufferung) des pH-Wertes; beispielsweise schlägt die WO 97/15329 hierzu vor, Dinatriumhydrogenphosphat zur pH-Stabilisierung zuzusetzen.

Für den Wirkstoff Dopamin wird in der WO 97/15329 jedoch keine Lösung des Problems angegeben, da sich die WO 97/15329 allein auf Zusammensetzungen mit Dobutamin als Wirkstoff bezieht und keine Angaben zu anderen Wirkstoffen enthält. Darüber hinaus wurde anmelderseits gefunden, daß wäßrige Lösungen mit Dopamin als Wirkstoff sich überraschenderweise - im Gegensatz zum Vorschlag gemäß WO 97/15329 für Dobutamin - durch Ascorbinsäure-Zusatz nicht ausreichend stabilisieren lassen.

Die US 5,002,973 beschreibt bereits Katecholamin-Lösungen für physiologische Verwendung, die einen pH-Wert im Bereich von 1,0 bis 5,0 aufweisen, und neben dem Katecholamin noch Acetylcystein, chelatisierende und puffernde Agentien enthalten. Diese Zusammensetzungen sind durch Acetylcystein bereits gegen Oxidation stabilisiert, vermeiden Sulfite und werden bevorzugt durch Inhalation verabreicht. Eine ausreichende Stabilisierung wird gemaß US 5,002,973 jedoch nur durch vergleichsweise hohe Konzentrationen des Stabilisators Acetylcystein erreicht, wobei diese Konzentration 0,05 bis 2,0 % w/v beträgt. Es ist wünschenswert, Acetylcystein durch Stabilisatoren zu ersetzen, die bereits in geringerer Konzentration, d.h. unterhalb der in der US 5.002,973 angegebenen Mindestkonzentration, eine effiziente Stabilisierung gewährleisten können.

Der vorliegenden Erfindung liegt daher die Aufgabe zugrunde, stabile wäßrige pharmazeutische Zusammensetzungen, enthaltend insbesondere Dopamin oder Dobutamin, bereitzustellen, die die Verwendung von Sulfiten vermeiden, aber dennoch die erfoderlichen Eigenschaften im Hinblick auf die Hitzesterilisation und Lagerstabilität aufweisen.

Zur Lösung der Aufgabe schlägt die Erfindung daher stabile flüssige pharmazeutische Zusammensetzungen vor, die einen der Wirkstoffe Dopamin oder Dobutamin, insbesondere Dopamin, und weiterhin eine zur Stabilisierung ausreichende Menge von 1.5 bis 6 mg Cystein-Hydrochlorid-Monohydrat pro 50 ml der Zusammensetzung und weiterhin wenigstens eine komplexbildende Hydroxycarbonsäure und/oder Mononatrium-Edetat enthalten, wobei der pH-Wert zwischen 3 ein 4 eingestellt ist. Zweckmäßig sind solche Zusammensetzungen die isotonische Zusammensetzungen für Injektionszwecke, insbesondere für die Anwendung in der Intensivmedizin, darstellen. Gegebenenfalls können die Zusammensetzungen bei höheren Wirkstoffkonzentrationen aber auch hyperton sein; oder, falls die zur Einstellung der Isotonie benötigte Menge NaCl zur Wirkstoffaussalzung führen würde (z.B. bei Konzentrationen von 250 mg Dobutamin in 20 ml), können die Zusammensetzungen auch hypoton sein.

Cystein ist eine natürliche Aminosäure, die auch im menschlichen Organismus vorkommt und deren Verwendung daher unbedenklich ist. Cystein kann als L-Cystein, D-Cystein oder D,L-Cystein vorliegen, sowie auch als Salz, insbesondere als Natriumsalz. Ferner kann Cystein als Cystein-Derivat als vorliegen, insbesondere pharmakologisch unbedenkliches Derivat. Solche pharmakologisch verträglichen Cystein-Derivate können z.B. Säureadditionssalze und/oder Hydrate sein, beispielsweise das Säureadditionssalz Cystein-Hydrochlorid. Als Cystein-Derivat wird in Rahmen der vorligenden Erfindung das Cystein-Hydrochlorid-Monohydrat (Cystein-HCl-H₂O) eingesetzt.

In den erfindungsgemäßen pharmazeutischen Zusammensetzungen wird das genannte Cystein-Derivat in einer auf das Volumen der Zusammensetzung bezogenen Menge von mehr als 1,5 mg pro 50 ml Zusammensetzung eingesetzt. Vorzugsweise beträgt die zur ausreichenden Stabilisierung des eingesetzten Wirkstoffes eingesetzte Menge an diesem Cystein-Derivat mehr als 2,5 mg pro 50 ml Zusammensetzung. Bezogen auf ein Volumen von 50 ml Zusammensetzung ist daher das Cystein-Hydrochlorid-Monohydrat in den erfindungsgemäßen pharmazeutischen Zusammensetzungen insgesamt in einer Menge von 1,5 bis 6 mg enthalten; vorzugsweise beträgt die Menge des Cystein und/oder Cystein-Derivates in den erfindungsgemäßen Zusammensetzungen 2,5 bis 3,5 mg pro 50 ml Zusammensetzung. Unter Berücksichtigung von Produktionszuschlägen und Laufzeitspezifikationen beträgt die Menge des eingesetzten Cystein-Derivates daher im allgemeinen, bezogen auf die gesamte Zusammensetzung etwa 0,003 Gew.-% bis 0,02 Gew.-%. Gegebenenfalls können auch höhere Mengen des genannten Cystein-Derivates eingesetzt werden, da Cystein und dessen pharmakologisch unbedenkliche Derivate für den Menschen nicht toxisch sind. Dennoch bewegt sich vorzugsweise die Menge an Cystein-Hydrochlorid-Monohydrat im vorstehend angegebenen Mengenbereich, der für Zwecke der Stabilisierung flüssiger wäßriger Dopamin- bzw. Dobutamin-Zusammensetzungen ausreichend ist.

Die pharmazeutischen Zusammensetzungen gemäß der Erfindung neben den angegebenen Wirkstoffen Dopamin oder Dobutamin und dem Stabilisator Cystein-Hydrochlorid-Monohydrat noch weitere Komponenten enthalten. In einer Variante der Erfindung können die Zusammensetzungen daher weiterhin eine komplexbildende Hydroxycarbonsäure, z.B. aus der Gruppe Citronensäure, Weinsäure, Äpfelsäure, Maleinsäure, Milchsäure, Gluconsäure, u.a. physiologisch verträgliche, in gewissem Ausmaß zur Komplexbildung befähigte Hydroxycarbonsäuren enthalten. Bevorzugt ist Citronensäure. Die Hydroxycarbonsäuren können auch in Form eines ihrer Salze bzw. Hydrate eingesetzt werden. Ein geeignetes Hydroxycarbonsäure-Monohydrat ist z.B. Citronensäure-Monohydrat (Citronensäure-H₂O). In einer weiteren Variante der Erfindung können die Zusammensetzungen, entweder zusätzlich oder anstelle der komplexierenden Hydroxycarbonsäure, auch einen pharmakologisch verträglichen Chelatbildner in geringer Menge enthalten, z.B. EDTA (Ethylendiamintetraessigsäure) oder EGTA (Ethylenglykol-bis(β-aminoethylether)-tetraessigsäure), insbesondere in Form der Natriumsalze. Beispielsweise enthalten die Zusammensetzungen in einer Variante der Erfindung Mononatrium-Edetat (Ethylendiamin-tetraessigsäure-Mononatriumsalz), das in einer Menge von bis zu 0,1 mg bezogen auf 1 ml der vollständigen flüssigen pharmazeutischen Zusammensetzung enthalten sein kann.

Der pH-Wert der erfindungsgemäßen Zusammensetzungen kann in an sich üblicher Weise auf den gewünschten pH-Wert zwischen 3 ber 4 eingestellt werden.

In speziellen Ausgestaltungen der Erfindung weisen die stabilen flüssigen pharmazeutischen Zusammensetzungen daher folgende Zusammensetzung auf:
- 5 bis 25 mg Dopamin-HCl oder Dobutamin-HCl,
- 0,03 bis 0,2 mg, vorzugsweise 0,06 bis 0,13 mg Cystein-HCl-H₂O,
- 7,3 bis 2,84 mg Natriumchlorid (zur Einstellung isotonischer Verhältnisse),
- gegebenenfalls bis zu 0,6 mg Zitronensäure oder Zitronensäure-Monohydrat,
- gegebenenfalls bis zu 0,1 mg Mononatrium-Edetat,
- gegebenfalls Natriumhydroxid zur Einstellung des pH-Wertes auf pH = 3 bis 4,
- Wasser für Injektionszwecke ad 1 ml.

Die erfindungsgemäßen Zusammensetzungen lassen sich in der für Injektionslösungen üblichen Weise durch Auflösen der Komponenten in Wasser für Injektionszwecke herstellen. In der Regel werden hierbei die Ansätze und auch die Lösungen nach Überführung in Ampullen durch ein geeignetes Inertgas, z.B. aus der Gruppe Stickstoff, Argon und Kohlendioxid, inertisiert; bevorzugtes Inertags ist hierbei Stickstoff. Die verschlossenen Ampullen werden üblicherweise noch einer Hitzesterilisation unterworfen.

Durch die Erfindung werden neue, vorteilhafte stabile wäßrig-flüssige pharmazeutische Zusammensetzungen, insbesondere für Dopamin, aber auch für Dobutamin bereitgestellt. Die erfindungsgemäß mit Cystein-Hydrochlorid-Monohydrat stabilisierten Zusammensetzungen vermeiden die Anwendung von Sulfiten als Stabilisator und gewährleisten dennoch eine ausreichende Stabilisierung der Zusammensetzungen. Dies gilt sowohl im Hinblick auf die Hitzesterilisation als auch die Lagerstabilität. Überraschenderweise genügen bereits sehr geringe Mengen an Cystein-Hydrochlorid-Monohydrat für eine ausreichende Stabilisierung der wäßrigen pharmazeutischen Zusammensetzungen mit den Wirkstoffen Dopamin oder Dobutamin.

Die nachfolgenden Beispiele sollen die Erfindung weiter erläutern, ohne sie jedoch in ihrem Umfange einzuschränken.

### Beispiele

### Beispiel 1:

### Rezepturbeispiele für Zusammensetzungen mit Dopamin-Hydrochlorid

Es wurden die in der nachfolgenden Tabelle angegebenen wäßrig-flüssigen pharmazeutischen Zusammensetzungen für Injektionszwecke hergestellt (alle Mengenangaben in mg/ml):

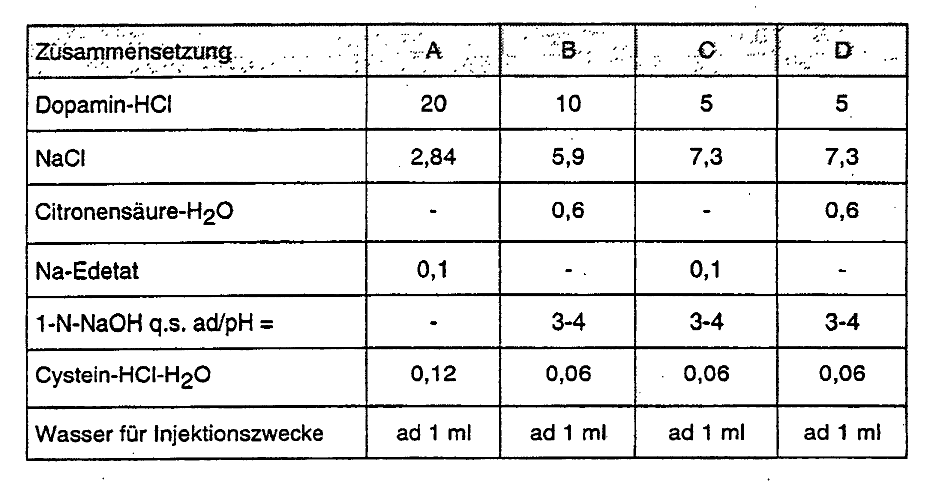

Die Ampullen mit den Zusammensetzungen sind bei Raumtemperatur gelagert mindestens 3 Monate stabil, d.h. sie zeigen keine Verfärbung. Die Zusammensetzungen sind auch bei Temperaturbelastung sehr stabil. So zeigt die Zusammensetzung C bei 100 °C erst nach 7 Tagen eine leichte Gelbtönung auf. Die Zusammensetzung D ist bei Lagerung bei 100 °C nach 9 Tagen noch immer farblos und klar. Die Zusammensetzungen C und D wurden auch auf ihre Kompatilibität mit Glucose getestet. Diese Zusammensetzungen sind z.B. mit 5 Gew.-% Glucose kompatibel und es tritt in Gegenwart von Glucose für mindestens 2 Tage keine Färbung auf.

### Beispiel 2:

### Rezepturbeispiel für eine Zusammensetzung mit Dobutamin-Hydrochlorid

Es wurde die in der nachfolgenden Tabelle angegebene wäßrig-flüssige pharmazeutische Zusammensetzung für Injektionszwecke hergestellt (alle Mengenangaben in mg/ml) :

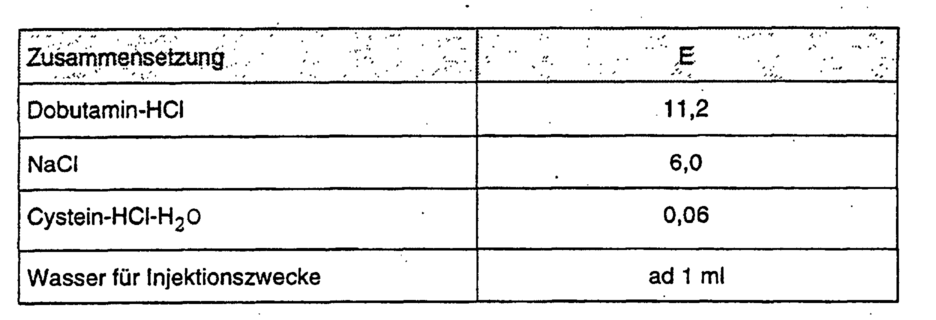

Die Ampullen mit der Zusammensetzung E sind bei Raumtemperatur gelagert für mindestens 2 Monate stabil, d.h. sie zeigen keine Verfärbung. Lagert man die Zusammensetzung unter Temperaturbelastung von 100 °C über einen Zeitraum von 24 Stunden, so wird nur eine sehr schwache Gelbtönung beobachtet.

### Vergleichsbeispiel 3:

### Vergleichszusammensetzungen von Dopamin-Hydrochlorid mit Ascorbinsäure als Stabilisator

Es wurden die in der nachfolgenden Tabelle angegebenen wäßrig-flüssigen pharmazeutischen Zusammensetzungen für Injektionszwecke zu Vergleichszwecken (siehe Stabilitätsuntersuchungen im Beispiel 4b) hergestellt (alle Mengenangaben in mg/ml):

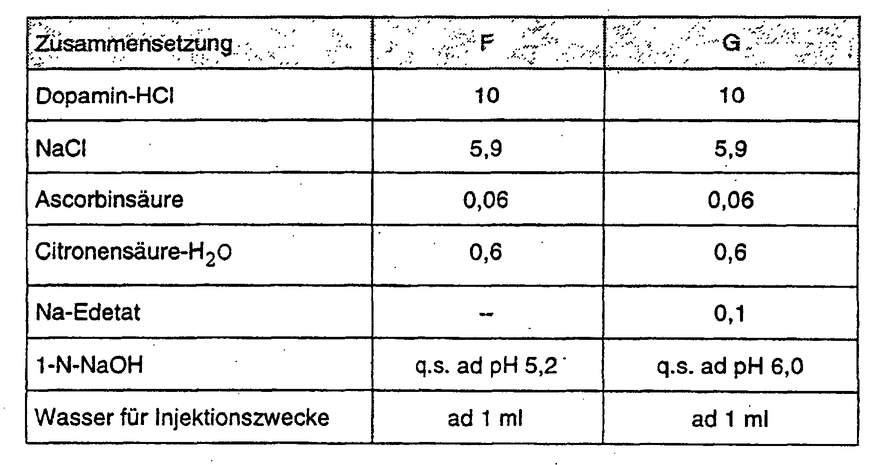

### Beispiel 4:

### Stabilitätsuntersuchungen an erfindungsgemäßen Zusamensetzungen (Beispiel 4.a) und an Vergleichszusammensetzungen (Beispiel 4.b)

Prüfung der Färbung z.B. gemäß Deutschem Arzneibuch (DAB): Die Prüfung der Farbstärke der Zusammensetzungen wurde im Bereich der Farben B (braun), BG (bräunlich-gelb),G (gelb) und GG (grünlich-gelb) wie folgt durchgeführt:

2,0 ml der zu prüfenden Zusammensetzung wurden mit 2,0 ml Wasser oder gemäß DAB vorgeschriebenen Farbvergleichslösung (siehe Gruppen B, BG, G, GG) in identischen, farblosen, durchsichtigen Reagenzgläsern aus Neutralglas von 12 mm äußerem Durchmesser verglichen. Die Beurteilung erfolgte bei diffusem Tageslicht in horizontaler Durchsicht gegen einen weißen Hintergrund.
- B9 =: farblos, d.h. die Flüssigkeit hat das Aussehen von Wasser und ist nicht stärker gefärbt als die Vergleichslösung BG des DAB
- <G7 =: ist gerade nicht mehr farblos
- G6 bis G4 =: leichte bis zunehmende Verfärbung gemäß Referenzlösungen DAB

Es ist zu beachten, daß die gegebenenfalls beobachteten Färbungen vom pH-Wert der Zusammensetzung beeinflußt sein können.
**a) Erfindungsgemäße Zusammensetzung:**
   Dopamin-Infusionslösungskonzentrat,
   10-ml-Ampullen aus farblosem Glas,
   hydrolytische Klasse I,
   Zusammensetzung A (gemäß Beispiel 1) 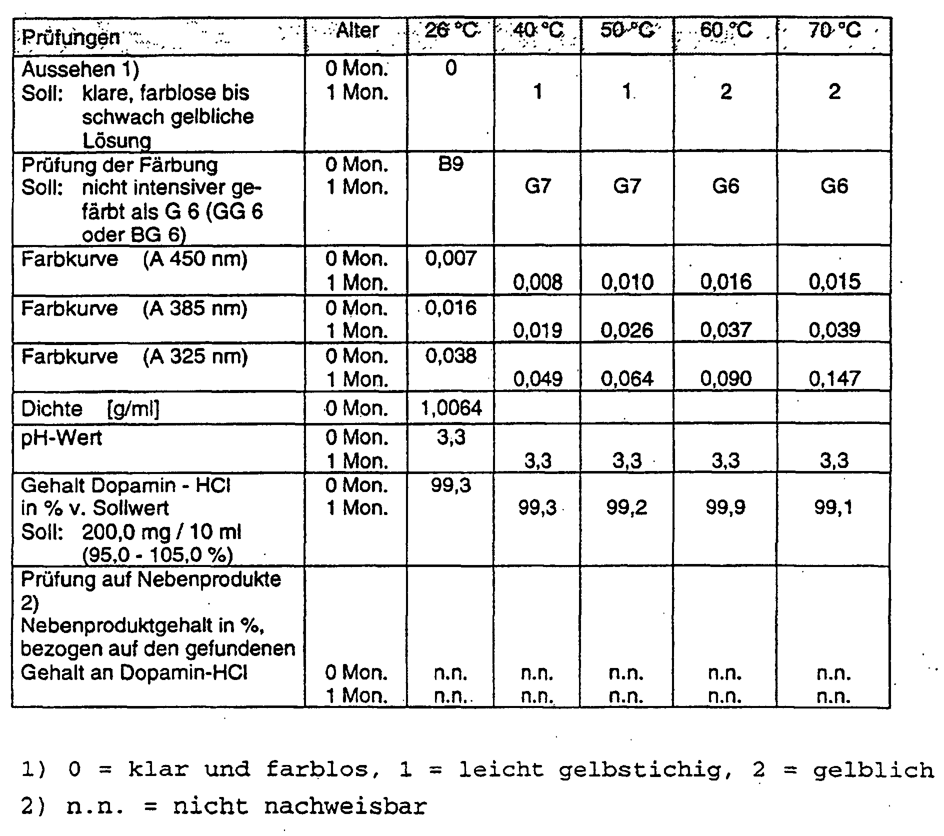
**b) Vergleichzusammensetzung mit Ascorbinsäure als Stabilisator:**
   Dopamin-Infusönslösungskonzentrat,
   10-ml-Ampullen aus farblosem Glas,
   hydrolytische Klasse I,
   Zusammensetzung F (gemäß Vergleichsbeispiel 3)

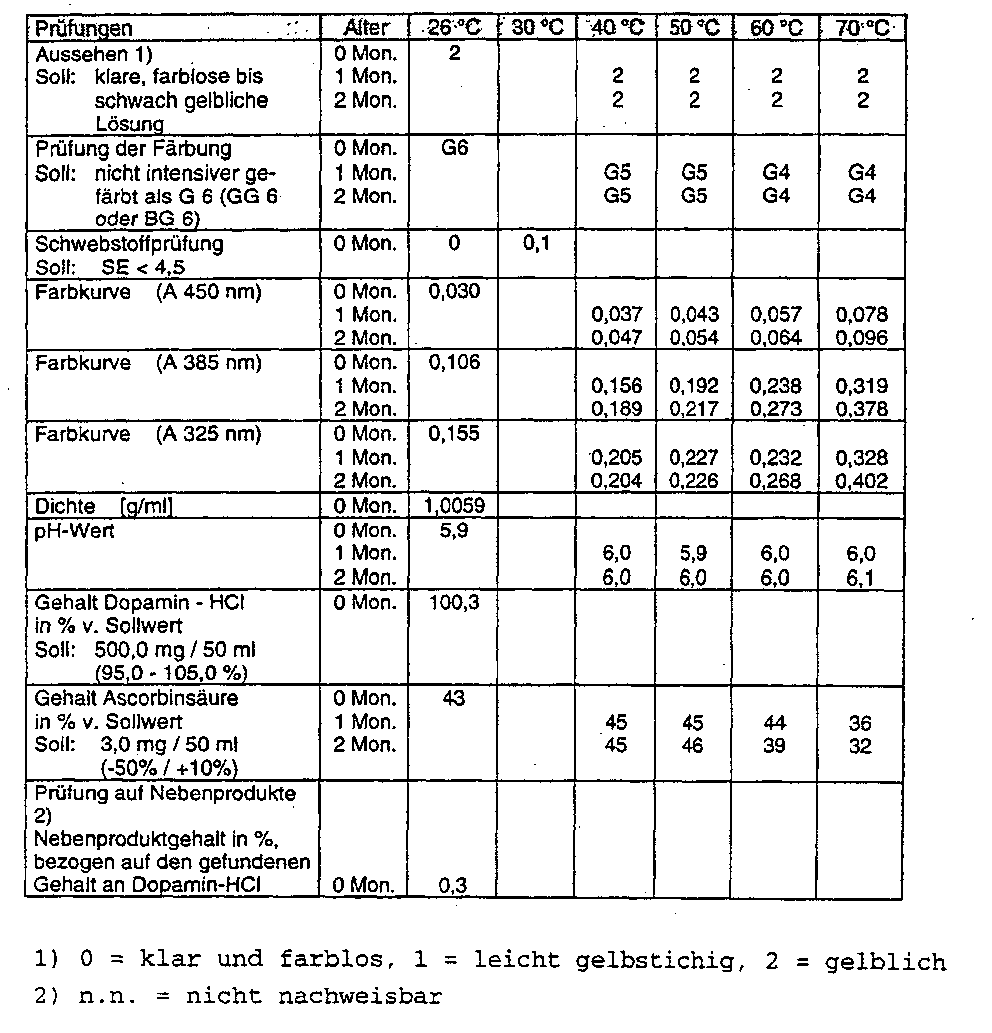

## Patentansprüche

1. Stabile wäßrig-flüssige pharmazeutische Zusammensetzung enthaltend einen der Wirkstoffe Dopamin oder Dobutamin und zur Stabilisierung Cystein-Hydrochlorid-Monohydrat in einer Menge von 1,5 bis 6 mg pro 50 ml der Zusammensetzung und weiterhin wenigstens eine komplexbildende Hydroxycarbonsäure und/oder Mononatrium-Edetat, wobei der pH-Wert zwischen 3 bis 4 eingestellt ist.

2. Pharmazeutische Zusammensetzung nach Anspruch 1, **dadurch gekennzeichnet, daß** das Cystein-Hydrochlorid-Monohydrat in einer Menge von 2,5 mg bis 3,5 mg pro 50 ml der Zusammensetzung eingesetzt wird.

3. Pharmazeutische Zusammensetzung nach Anspruch 1 oder 2, **dadurch gekennzeichnet, daß** als komplexbildende Hydroxycarbonsäure Citronensäure oder deren Hydrate, enthalten sind.

4. Pharmazeutische Zusammensetzung nach einem der vorhergehenden Ansprüche, **dadurch gekennzeichnet, daß** sie eine komplexbildende Hydroxycarbonsäure und Mononatrium-Edetat enthält.

5. Pharmazeutische Zusammensetzung nach einem der vorhergehenden Ansprüche, **dadurch gekennzeichnet, daß** sie eine isotonische Zusammensetzung für Injektionszwecke ist.

6. Pharmazeutische Zusammensetzung nach einem der vorhergehenden Ansprüche mit folgender Zusammensetzung:
- 5 bis 25 mg Dopamin-HCl oder Dobutamin-HCl,
- 0,03 bis 0,2 mg Cystein-HCl-H₂O,
- 7,3 bis 2,84 mg Natriumchlorid,
- bis zu 0,6 mg Citronensäure oder Citronensäure-Monohydrat und/oder bis zu 0,1 mg Mononatrium-Edetat,
- gegebenfalls Natriumhydroxid zur Einstellung des pH-Wertes auf pH = 3 bis 4,
- Wasser für Injektionszwecke ad 1 ml.

## Claims

1. A stable aqueous-liquid pharmaceutical composition containing one of the active substances dopamine or dobutamine and for stabilisation cysteine hydrochloride monohydrate in a quantity of 1.5 to 6 mg per 50 ml of the composition and furthermore at least one complexing hydroxycarboxylic acid and/or monosodium edetate, the pH being set to between 3 and 4.

2. A pharmaceutical composition according to Claim 1, **characterised in that** the cysteine hydrochloride monohydrate is used in a quantity of 2.5 mg to 3.5 mg per 50 ml of the composition.

3. A pharmaceutical composition according to Claim 1 or 2, **characterised in that** citric acid or hydrates thereof is contained therein as the complexing hydroxycarboxylic acid.

4. A pharmaceutical composition according to one of the preceding claims, **characterised in that** it contains a complexing hydroxycarboxylic acid and monosodium edetate.

5. A pharmaceutical composition according to one of the preceding claims, **characterised in that** it is an isotonic composition for injection purposes.

6. A pharmaceutical composition according to one of the preceding claims, having the following composition:
- 5 to 25 mg dopamine-HCl or dobutamine-HCl,
- 0.03 to 0.2 mg cysteine-HCl-H₂O,
- 7.3 to 2.84 mg sodium chloride,
- up to 0.6 mg citric acid or citric acid monohydrate and/or up to 0.1 mg monosodium edetate,
- optionally sodium hydroxide to set the pH to pH = 3 to 4,
- water for injection purposes ad 1 ml.

## Revendications

1. Composition pharmaceutique liquide aqueuse stable contenant l'un des principes actifs dopamine ou dobutamine et, pour stabiliser, du chlorhydrate de cystéine monohydraté dans une quantité comprise entre 1,5 et 6 mg pour 50 ml de la composition, et en outre au moins un acide hydroxycarboxylique complexant et/ou un édétate monosodique, la valeur du pH étant ajustée entre 3 et 4.

2. Composition pharmaceutique selon la revendication 1, **caractérisée en ce que** le chlorhydrate de cystéine monohydraté est utilisé dans une quantité comprise entre 2,5 mg et 3,5 mg pour 50 ml de la composition.

3. Composition pharmaceutique selon la revendication 1 ou 2, **caractérisée en ce qu'**elle contient de l'acide citrique ou ses hydrates en tant qu'acide hydroxycarboxylique complexant.

4. Composition pharmaceutique selon l'une quelconque des revendications précédentes, **caractérisée en ce qu'**elle contient un acide hydroxycarboxylique complexant et de l'édétate monosodique.

5. Composition pharmaceutique selon l'une quelconque des revendications précédentes, **caractérisée en ce qu'**elle est une composition isotonique utilisée à des fins d'injection.

6. Composition pharmaceutique selon l'une quelconque des revendications précédentes, constituée de :
- 5 à 25 mg de dopamine-HCl ou de dobutamine-HCl,
- 0,03 à 0,2 mg de cystéine-HCl-H₂O,
- 7,3 à 2,84 mg de chlorure de sodium,
- jusqu'à 0,6 mg inclus d'acide citrique ou d'acide citrique monohydraté et/ou jusqu'à 0,1 mg inclus d'édétate monosodique,
- éventuellement d'hydroxyde de sodium pour l'ajustement de la valeur du pH à pH = 3 à 4,
- d'eau pour réaliser les injections, jusqu'à 1 ml.
